(19) Europäisches Patentamt
European Patent Office
Office européen des brevets

(11) Numéro de publication: 0 210 886
B1

(12) **FASCICULE DE BREVET EUROPEEN**

(45) Date de publication du fascicule du brevet: **24.08.88**

(51) Int. Cl.⁴: **C 07 C 33/46**, A 61 K 31/065, C 07 C 29/40

(21) Numéro de dépôt: **86401369.3**

(22) Date de dépôt: **20.06.86**

(54) **Alcools tertiaires halogéno biphéniles utiles en thérapeutique dans le traitement de l'athérosclérose.**

(30) Priorité: **26.07.85 FR 8511582**

(43) Date de publication de la demande: **04.02.87 Bulletin 87/6**

(45) Mention de la délivrance du brevet: **24.08.88 Bulletin 88/34**

(84) Etats contractants désignés: **AT BE CH DE GB IT LI LU NL SE**

(56) Documents cités:
**FR - A - 2 208 673**
**US - A - 4 053 639**

(73) Titulaire: **P.F. MEDICAMENT, 125, Rue de la Faisanderie, F-75116 Paris (FR)**

(72) Inventeur: **Cousse, Henri La Foun de los Nobios, Chemin de Lastinos, F-81100 Castres (FR)**
Inventeur: **Delhon, André, 36, rue d'Hector Berlioz, F-81100 Castres (FR)**
Inventeur: **Rieu, Jean-Pierre La Vixère Haute, Avenue du Sidobre, F-81100 Castres (FR)**
Inventeur: **Patoiseau, Jean-François, 7, rue Jules Ferry, F-81100 Castres (FR)**

(74) Mandataire: **Doat, Jean-Pierre, PIERRE FABRE S.A. Service Propriété Industrielle 17, Avenue Jean Moulin, F-81106 Castres Cédex (FR)**

EP 0 210 886 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés d'alcools tertiaires halogéno biphényles possédant des propriétés hypolipémiante, hypocholestérolémiante et anorexigène, leur procédé de préparation et leur application en tant que médicaments utiles dans le traitement et la prévention des troubles de l'athérosclérose et de l'obésité.

Dans le brevet FR-A-2.208.673 (Karl Tomae), il est notamment question de 2-(2'-fluoro-4-biphénylyl)-propène ou butène obtenus à partir des carbinols correspondants, ayant un effet antiphlogistique.

En outre, le brevet US-A-4.053.639 (ST.S. Adams), concerne notamment des 2-(2'-fluoro-4-biphénylyl)propan-1-ol ayant une activité anti-inflammatoire, analgésique et antipyrétique.

Dans les brevets FR 2.476.072 et FR 2.498.449 déposés par la titulaire, il est fait état de dérivés de l'acide halogénobiphényl carboxylique utiles dans le traitement des troubles provoqués par l'athérosclérose.

Les nouveaux composés chimiques découverts par la titulaire présentent une originalité structurale par la présence du goupe fonctionnel alcool tertiaire.

La présente invention a pour objet des alcools tertiaires halogéno biphényles de formule générale (I):

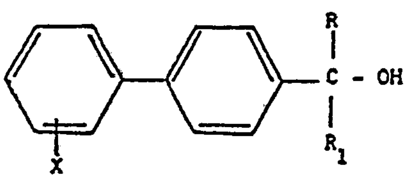

(I)

dans laquelle:

X représente un atome d'halogène chlore, ou brome en position ortho ou méta.

R et $R_1$ identiques ou différents représentent un groupe alcoyle de bas poids moléculaire de 1 à 4 atomes de carbone.

La présente invention concerne également un procédé de préparation des dérivés de formule I par réaction des cétones de formule II:

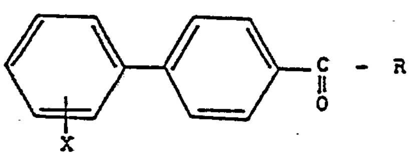

(II)

dans laquelle X a la même signification que dans la formule I avec un dérivé magnésien de formule $R_1$-$Mg_Y$ (Y=iode, brome ou chlore), selon le schéma réactionnel:

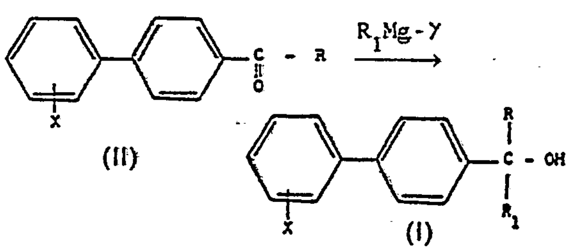


La présente invention concerne de même l'utilisation des composés de formule I à titre de médicaments ainsi que les compositions pharmaceutiques contenant ces médicaments. Les compositions pharmaceutiques selon la présente invention peuvent comporter un ou plusieurs composés de formule I éventuellement en association avec d'autres principes actifs. Parmi les dérivés de formule I, on peut citer plus particulièrement les dérivés suivants:

– (chloro-2' biphényl-4 yl) diméthyl carbinol (F 2833)
– (chloro-2' biphényl-4 yl) éthyl méthyl carbinol (F 2883)
– (chloro-2' biphényl-4 yl) diéthyl carbinol (F 2884)
– (chloro-2' biphényl-4 yl) isopropylméthyl carbinol (F 2885)
– (chloro-3' biphényl-4 yl) diméthyl carbinol (F 2886)
– (bromo-2' biphényl-4 yl) diméthyl carbinol

*Exemple 1:*

*(Chloro-2' biphényl-4 yl) diméthylcarbinol (F 2833)*

Une solution de magnésium de l'iodure de méthyle est préparée à partir de magnésium (2,53 g) et d'iodure de méthyle 7 ml (16,24 g, 0,114 mole) dans l'éther éthylique (100 ml).

A cette préparation, on ajoute goutte à goutte une solution de chloro-2' acétyl-4 biphényle (12 g, 52 mmoles) dans un mélange éther/benzène (60 ml/60 ml).

Après une heure de reflux, le mélange est ramené à température ambiante et hydrolysé par une solution saturée de chlorure d'ammonium (20 ml, 0,09 mole).

La phase organique séparée est lavée à l'eau (100 ml) au bicarbonate (100 ml) puis à l'eau salée (100 ml).

La solution séchée sur carbonate de sodium, la solution est concentrée sous vide à une température n'excédant pas 40° C.

Par reprise dans l'heptane (100 ml) et refroidissement on obtient avec un rendement de 30% le dérivé alcool tertiaire.

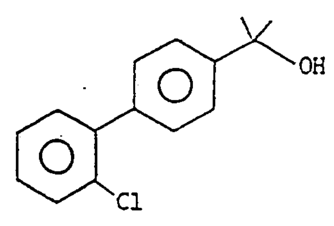

Formule brute: $C_{15}H_{15}ClO$
Masse moléculaire: 246,74
Point de fusion: 55° C
Chromatographie sur couche mince:
  support: gel de silice 60 F 254 Merck
  solvant: hexane - acétate d'éthyle
  révélation: UV
  Rf: 0,70
Solubilité: 1% dans le propylène glycol.

D'une manière analogue à celle décrite à l'exemple 1, et en partant des dérivés halogénés et de la cétone appropriés, les composés suivants ont été préparés.

*Exemple 2:*

*(Chloro-2' biphényl-4 yl) éthyl, méthyl carbinol (F 2883)* (obtenu en utilisant l'iodure d'éthyle)

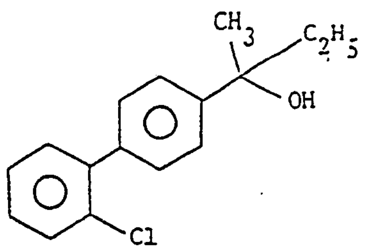

Formule brute: $C_{16}H_{17}ClO$
Masse moléculaire: 260,76
Huile incolore: Eb: 150° C ($10^{-3}$ mbar)
$n_D^{23}$: 1,5915
Chromatographie sur couche mince:
support: gel de silice 60 F 254 Merck
solvant: hexane - acétate d'éthyle 70/30
révélation: UV
Rf: 0,5
Solubilité: 5% dans propylène glycol

*Exemple 3:*

*(Chloro-2' biphényl-4 yl) diéthylcarbinol (F 2884)* (obtenu à partir de chloro-2' propionyl-4 biphényle et d'iodure d'éthyle)

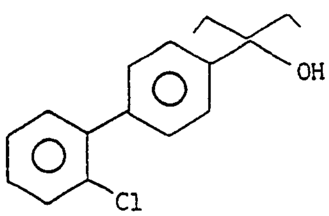

Formule brute: $C_{17}H_{19}Cl{-}O$
Masse moléculaire: 274,79
Huile incolore: Eb: 160° C ($10^{-3}$ bar)
$n_D^{23}$: 1,5843
Chromatographie sur couche mince:
support: gel de silice 60 F 254 Merck
solvant: hexane - acétate d'éthyle 70/30
révélation: UV
Rf: 0,60
Solubilité: 10% dans propylène glycol.

*Exemple 4:*

*(Chloro-2' biphényl-4 yl) isopropyl méthyl carbinol (F 2885)* (obtenu à partir de chloro-2' acétyl-4 biphényl et iodure d'isopropyle)

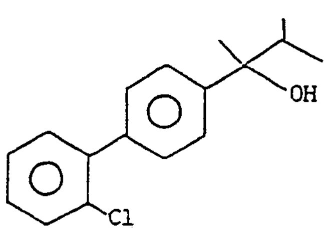

Formule brute: $C_{17}H_{19}ClO$
Masse moléculaire: 274,79
Huile jaune clair: Eb: 150° C ($10^{-3}$ mbar)
$N_D^{23}$: 1,5855
Chromatographie sur couche mince:
support: gel de silice 60 F 254 Merck
solvant: hexane - acétate d'éthyle 70/30
révélation: UV
Rf: 0,60
Solubilité: 10% dans propylène glycol.

*Exemple 5:*

*(Chloro-3' biphényl-4 yl) diméthyl carbinol (F 2886)* (obtenu à partir de chloro-3' acétyl-4 biphényle et iodure de méthyle)

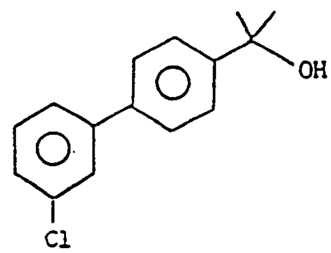

Formule brute: $C_{15}H_{15}ClO$
Masse moléculaire: 246,74
Point de fusion: 71° C
Chromatographie sur couche mince:
support: gel de silice 60 F 254 Merck
solvant: hexane - acétate d'éthyle 70/30
révélation: UV
Rf: 0,35
Solubilité: 2% dans propylène glycol

De façon analogue en partant de la cétone correspondante est obtenu:

*Exemple 6:*

*(bromo-2 biphényl-4 yl) diméthyl carbinol*

*Expérimentations*

Divers essais toxicologiques et pharmacologiques ont été effectués sur les composés objet de la présente invention

A) Toxicologie

Les composés de l'invention ont été soumis à des contrôles de toxicologie. Cette toxicité a été déterminée par la dose létale 50; elle a été recherchée sur des lots de 10 souris par voie orale et intra-veineuse et calculée selon la méthode de MILLER et TAINER (Proc. Soc. Exper. Biol. Med., 1944, 57, 261).

Les $DL_{50}$ des composés testés sont supérieures à 100 mg/kg par voie intra-veineuse et à 1000 mg/kg par voie orale.

B) Propriétés pharmacologiques

Les expérimentations pharmacologiques ont permis de mettre en évidence de remarquables propriétés hypolipémiante et hypocholestérolémiante.

Ci-après sont rapportés les résultats obtenus avec les produits des exemples 1 à 5 qui ont été comparés au CLOFIBRATE et au FENOFIBRATE.

Test portant sur 4 jours de traitement par voie orale selon la méthode de BUCHMAN, SPRANCMANIS et PARTYKA (J. Med. Chem. 12, 1001-1006, 1969). Rats mâles, naïfs, Sprague Dawley, répartis en lots homogènes de 8 animaux.

Traitement par voie orale par les produits à étudier en solution ou suspension dans la CMC 1% sous un volume de 10 ml/kg.

Durée du traitement: 4 jours.

Fréquence des traitements: 1 fois par jour.

Le cinquième jour, prélèvement de sang sur héparine à l'artère caudale après un jeûne de nourriture d'environ 16 heures.

Les résultats sont reportés dans le tableau suivant comparativement au CLOFIBRATE et au FENOFIBRATE.

| Produits | Dosage mg/kg/jour | Cholestérol % baisse par rapport au témoin | Consommation nourriture % variation par rapport au témoin |
|---|---|---|---|
| CLOFIBRATE | 100<br>200<br>300 | −17<br>− 8 (NS)<br>−12 (NS) | +14<br>−10<br>−40 |
| FENOFIBRATE | 100<br>200<br>300 | −16<br>−27<br>−25 | +23<br>+10<br>−17 |
| EXEMPLE 1 (F 2833) | 25<br>50<br>100 | −16<br>−31<br>−39 | −19<br>−20<br>−30 |
| EXEMPLE 2 (F 2883) | 100 | −28 | 0 |
| EXEMPLE 3 (F 2884) | | −41 | 0 |
| EXEMPLE 4 (F 2885) | | −10 | 0 |
| EXEMPLE 5 (F 2886) | | 0 | −40 |

C) Applications thérapeutiques

Compte tenu de leurs propriétés pharmacologiques, les composés de l'invention et plus particulièrement les composés des exemples 1, 2, 3 et 4 peuvent être utilisés en thérapeutique dans le traitement des différents types d'hyperlipidémies en vue de la prévention et du traitement de l'athérosclérose et de l'obésité. Les préparations pharmaceutiques contenant ces principes actifs peuvent être administrées par voie orale ou parentérale. Il est également possible d'y associer d'autres principes actifs pharmaceutiquement et thérapeutiquement acceptables.

## Revendications

1. A titre de composés chimiques nouveaux, les alcools tertiaires halogènes biphényles répondant à la formule générale (I):

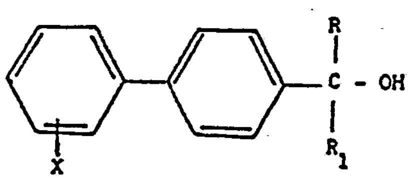 (I)

dans laquelle:

X représente un atome d'halogène, chlore ou brome, en position ortho ou méta.

R et $R_1$ sont identiques ou différents et représentent un groupe alcoyle de bas poids moléculaire contenant de 1 à 4 atomes de carbone.

2. Un composé selon la revendication 1, caractérisé en ce qu'il est choisi parmi:
– (chloro-2' biphényl-4 yl) diméthyl carbinol (F 2833)
– (chloro-2' biphényl-4 yl) éthyl méthyl carbinol (F 2883)
– (chloro-2' biphényl-4 yl) diéthyl carbinol (F 2884)
– (chloro-2' biphényl-4 yl) isopropyl méthyl carbinol (F 2885)
– (chloro-3' biphényl-4 yl) diméthyl carbinol (F 2886)
– (bromo-2' biphényl-4 yl) diméthyl carbinol.

3. Procédé de préparation des composés chimiques selon les revendications 1 et 2, caractérisé en ce que l'on traite un dérivé cétonique de formule (II):

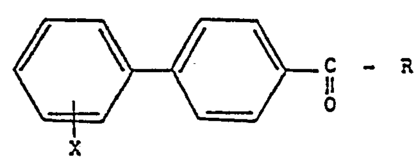 (II)

par un organomagnésien de formule $R_1MgY$ pour obtenir les composés de formule générale (I).

X, R et $R_1$ ayant les significations données à la revendication 1,

Y représentant un halogène (iode, brome ou chlore).

4. A titre de médicaments nouveaux utiles en thérapeutique, notamment dans le traitement de l'obésité et des troubles provoqués par l'athérosclérose, les produits selon les revendications 1 et 2.

5. Compositions pharmaceutiques, caractérisées en ce qu'elles contiennent comme principe actif au moins un dérivé selon l'une des revendications 1 et 2.

6. Compositions pharmaceutiques selon la revendication 5, caractérisées en ce qu'elles sont administrées par voie orale ou parentérale.

7. Compositions pharmaceutiques selon les revendications 5 et 6, caractérisées en ce qu'elles contiennent d'autres principes actifs associés aux composés chimiques des revendications 1 et 2.

## Claims

1. As novel chemical compounds, the halogenated biphenyl tertiary alcohols corresponding to the general formula (I):

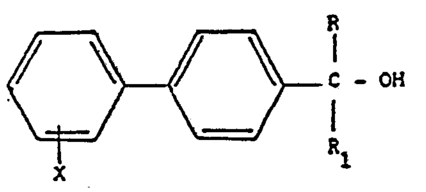

(I)

in which:

X represents a halogen atom, chlorine or bromine, in the *ortho* or *meta* position, and

R and $R_1$ are identical or different and represent a low-molecular-weight alkyl group containing from 1 to 4 carbon atoms.

2. A compound according to claim 1, characterised in that it is selected from:
– 2'-chloro-4-biphenylyl dimethyl carbinol (F 2833)
– 2'-chloro-4-biphenylyl ethyl methyl carbinol (F 2883)
– 2'-chloro-4-biphenylyl diethyl carbinol (F 2884)
– 2'-chloro-4-biphenylyl isopropyl methyl carbinol (F 2885)
– 3'-chloro-4-biphenylyl dimethyl carbinol (F 2886)
– 2'-bromo-4-biphenylyl dimethyl carbinol.

3. Process for the preparation of the chemical compounds according to claims 1 and 2, characterised in that a ketone derivative of the formula (II):

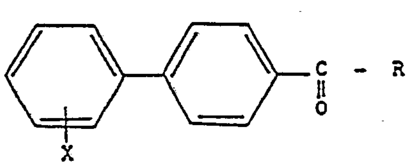

(II)

is treated with an organomagnesium derivative of the formula $R_1MgY$ in order to obtain the compounds of the general formula (I),

X, R and $R_1$ having the meanings given in claim 1, and Y representing a halogen atom (iodine, bromine or chlorine).

4. The products according to claims 1 and 2 for use as novel medicaments in therapy, especially in the treatment of obesity and disorders caused by atherosclerosis.

5. Pharmaceutical compositions, characterised in that they contain as active ingredient at least one derivative according to claim 1 or claim 2.

6. Pharmaceutical compositions according to claim 5, characterised in that they are administered orally or parenterally.

7. Pharmaceutical compositions according to claims 5 and 6, characterised in that they contain other active ingredients in conjunction with the chemical compounds of claims 1 and 2.

## Patentansprüche

Tertiäre halogenierte Biphenylalkohole der allgemeinen Formel (I):

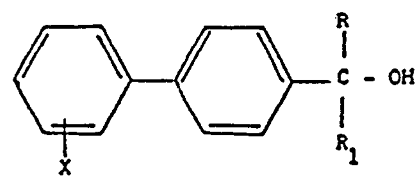

(I)

worin

X eines der Halogenatome Chlor oder Brom in ortho- oder meta-Stellung bedeutet,

R und $R_1$ gleich oder verschieden sind und eine Alkylgruppe mit niedrigem Molekulargewicht und 1 bis 4 Kohlenstoffatomen darstellen, als neue chemische Verbindungen.

2. Verbindungen nach Anspruch 1, dadurch gekennzeichnet, dass sie ausgewählt sind aus:
(2'-Chlor-biphenyl-4 yl) dimethylcarbinol (F 2833)
(2'-Chlor-biphenyl-4 yl) ethyl methylcarbinol (F 2883)
(2'-chlor-biphenyl-4 yl) diethylcarbinol (F 2884)
(2'-Chlor-biphenyl-4 yl) isopropylmethylcarbinol (F 2885)
(3'-Chlor-biphenyl-4 yl) dimethylcarbinol (F 2886)
(2'-Brom-biphenyl-4 yl) dimethylcarbinol.

3. Verfahren zur Herstellung von chemischen Verbindungen nach den Ansprüchen 1 und 2, dadurch gekennzeichnet, dass man ein Ketonderivat der Formel (II):

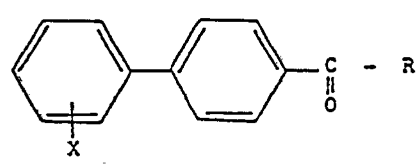

(II)

mit einer Organomagnesiumverbindung der Formel $R_1MgY$, wobei in den Formeln X, R und $R_1$ die in Anspruch 1 angegebene Bedeutung haben und

Y ein Halogenatom (Jod, Brom oder Chlor) bedeutet, behandelt, um eine Verbindung der allgemeinen Formel (I) zu erhalten.

4. Neue Arzneimittel, die zur Therapie insbesondere zur Behandlung der Fettleibigkeit und von durch Arteriosklerose hervorgerufenen Beschwerden, in Form von Produkten nach den Ansprüchen 1 und 2.

5. Pharmazeutische Zusammensetzungen, dadurch gekennzeichnet, dass sie als Wirkstoff mindestens ein Derivat nach einem der Ansprüche 1 und 2 enthalten.

6. Pharmazeutische Zusammensetzungen nach Anspruch 5, dadurch gekennzeichnet, dass sie oral oder parenteral verabreicht werden.

7. Pharmazeutische Zusammensetzungen nach den Ansprüchen 5 und 6, dadurch gekennzeichnet, dass sie weitere Wirkstoffe in Verbindung mit chemischen Verbindungen der Ansprüche 1 und 2 enthalten.